Europäisches Patentamt

European Patent Office

Office européen des brevets

(19)

(11) Publication number: **0 399 040 B1**

(12) **EUROPEAN PATENT SPECIFICATION**

(45) Date of publication of patent specification: 07.10.92

(21) Application number: 87902738.1

(22) Date of filing: 20.04.87

(86) International application number:
PCT/JP87/00249

(87) International publication number:
WO 87/06103 (22.10.87 87/23)

(51) Int. Cl.⁵: **A23L 1/20**, A23L 1/212,
A23L 1/28, A23L 1/304,
C12N 1/16

(54) **PROCESS FOR PRODUCING IRON-RICH FOODS.**

(30) Priority: 19.04.86 JP 90899/86
23.01.87 JP 13680/87

(43) Date of publication of application:
28.11.90 Bulletin 90/48

(45) Publication of the grant of the patent:
07.10.92 Bulletin 92/41

(84) Designated Contracting States:
BE DE FR GB IT SE

(56) References cited:
JP-A-54 157 890
JP-A-58 101 686
JP-B-25 650 816

(73) Proprietor: TERUMO KABUSHIKI KAISHA
No. 44-1, Hatagaya 2-chome, Shibuya-ku
Tokyo 151(JP)

(72) Inventor: ISHIGAKI, Reisaburo
14-43, Sannodai
Numazu-shi Shizuoka-ken 410(JP)

(74) Representative: Joly, Jean-Jacques et al
CABINET BEAU DE LOMENIE 55, rue
d'Amsterdam
F-75008 Paris(FR)

EP 0 399 040 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Technical Field

The present invention relates to a process for preparing iron enriched fermented food products.

Background Technology

From old times a dish of carrot, sea tangle ("Kombu") and soybean cooked together in an iron pan had widely been taken as a daily dish. Recently, however, the use of iron pot has become unpopular so that it is no longer a source of iron supply. In the preparation of soybean cake ("Miso") or soybean sauce ("Shoyu"), the starting soybean is boiled in an iron pot and then combined with rice malt (Koji) to be processed to "Miso" or combined with parched wheat to make "Koji" to be processed to "Shoyu". The iron which is derived from an iron pot used in the course of preparation of "Shoyu" or "Miso" is reacted with lactic acid produced during the fermentation process to be converted to an organic form or to be introduced into the yeast cell. However, iron that remains in organic form in fermented products causes deterioration of color of the products with elapse of time so that fermentation equipment in which iron is used in contact with the fermentation products has become out of use. Consequently, these fermentation products are also no longer a source of iron supply. On the other hand, mixtures of ferric citrate or ferrous lactate with a plant extract have heretofore been used for supply of iron but occasionally caused inflammation on or around the lips or anorexia.

We previously developed a process for preparing fermented food products of soybean by digesting boiled powdered raw soybean with digestive enzyme and then fermenting the digest with yeast, for which a patent application was filed (Japanese Patent Publication No. 50816/81). According to the preparative process there are produced fermented food products of soybean which are associated with none of unpleasant color and odor caused by growth of fungi and bacteria. Whereas soybean is a nutritious food rich in protein, lipid and saccharide, its iron content is as low as 10 mg or below per 100 g of edible part. As a result of extensive studies for reinforcing iron content in the above-mentioned fermented food products of soybean, we have found that the iron enriched food products with good flavor and high nutritive value are provided by digesting pulverized soybean and cultivating yeast in the digest in the presence of an iron compound.

There are also provided the iron enriched food products with good flavor and rich in carotene by digesting pulverized carrot and cultivating yeast in the digest in the presence of an iron compound.

Furthermore, there are provided the iron enriched food products with very high nutritive value and rich in iron in readily absorbable form by digesting pulverized soybean and pulverized carrot and cultivating yeast in the digest mixture in the presence of an iron compound.

Disclosure of the Invention

The present invention has been completed on the basis of the above findings and is constituted of:

1. A process for preparing an iron enriched food product which comprises hydrolyzing pulverized soybean, pulverized carrot or a mixture of the two with saccharide-decomposing enzyme and cultivating yeast in said hydrolyzate in the presence of an iron compound.

2. A process according to item 1 wherein pulverized and boiled soybean produced by mixing powdery raw soybean with sizes not larger than 200 mesh (74 $\mu$m) with hot water and boiling the mixture, said powder raw soybean being obtained by finely pulverizing skinned raw soybean, or boiled and pulverized carrot produced by boiling carrot and pulverizing the boiled carrot, or a mixture of the two is hydrolyzed with amylase, and Saccharomyces cerevisiae is cultivated in said hydrolyzate in the presence of an iron compound.

3. A process according to item 1 or 2 wherein the iron compound is iron citrate or iron lactate.

In carrying out the process of the invention, skinned raw soybean is finely pulverized to obtain fine powders with sizes of not larger than 200 mesh (74 $\mu$m). The above step facilitates dissolution of the water-soluble components of soybean, which in turn makes the soybean more sensitive to hydrolase. Preferably, the powdery raw soybean is mixed with hot water, and the mixture is rapidly boiled to inactivate enzymes in the powdery raw soybean. When carrot is employed, water is added to raw carrot, which is then wet pulverized. Preferably, carrot is cooked before pulverized. To the pulverized soybean or carrot, or a mixture of the two is added saccharide-decomposing enzyme to hydrolyze saccharides in the starting material such as starch and cellulose for saccharification. The starting soybean or carrot is used in an amount so as to give a saccharide concentration of 1-250 g/l after hydrolysis. Soybean at a level of 3-700 g/l, preferably 3-350 g/l, on dry basis or carrot at a level of 1.5-390 g/l, preferably 1.5-200 g/l, on dry basis is used alone or in appropriate combination. If soybean and carrot are employed in combination, the soybean is in an amount of 1-90 parts, preferably 1-50 parts per a total of 100 parts by weight (dry basis). As the saccharide-decomposing enzyme are employed amylases, cellulases or the like. As the amylase are used $\alpha$-amylase, $\beta$-amylase, glucoamylase, isoamylase and the like. The saccharization is conducted under such conditions as addition of an enzyme at a ratio of 1/1000-3/1000 (by weight) to the substrate, at a temperature of 10-50°C or 10-90°C for heat-resistant enzyme for a period of 10 min.-5 hours, these being variable depending upon activity of the enzyme. Saccharide concentration of the hydrolyzate is desirably in the range of about 1-250 g/l. After completion of the hydrolysis, an iron compound is added to the resulting hydrolyzate, and the mixture is innoculated with yeast followed by cultivation. The iron compound may be added before hydrolysis step, and the amount to be added (concentration in the culture) is desirably in the range of about 25-1500 mg/l. There is no particular limitation to nature of the iron compound, which may be an organic or inorganic ferrous or ferric compound. For example, ferric chloride, ferrous or ferric sulfate, iron citrate, iron tartrate, ferrous lactate, ferrous gluconate, ferrous or ferric pyrophosphate, ferric cholineisocitrate and the like are used. It is preferable to employ Saccharomyces cerevisiae as the yeast. Amount of the yeast used (concentration in the culture) is in the range of 5-300 g/l for raw yeast or 2-150 g/l for dry yeast. Cultivation is carried out usually at 5-50°C and preferably at 10-30°C for a period of 30 min. to 4 days.

It is preferred to neutralize organic acids which have been formed in the culture during the above-mentioned cultivation of yeast with calcium carbonate to a pH of approximately 5. The neutralization improves strong acid taste and also achieves enrichment of calcium. The culture thus obtained is used as it is or dried by a conventional method to yield an iron enriched food product. Freeze drying, spray drying, hot air drying, vacuum drying and the like may be adopted, and freeze drying is most preferable.

In the above-described process of the invention, the starting material is a digestion product of pulverized soybean, pulverized carrot or a mixture of the two so that introduction of iron content into yeast and formation of the complex are attained most effectively to result in good absorption of the iron content through the digestive tract.

Absorption ratio of iron through digestive tract is variable depending upon types of the iron. It greatly differs between inorganic iron and organic iron, and also between the organic iron existing in plant and the iron in animal widely existing as hem iron. Iron is absorbed as a divalent iron mainly through the region from the duodenum to the ileum. It is absorbed in the form of reduced iron through cells of the intestinal wall. The absorption ratio of iron is also variable depending upon moving rate of the iron in the intestinal tract.

Heretofore, ferric citrate or ferrous lactate has been used for iron enrichment in the form of a simple mixture with a plant extract. Although the iron is possibly absorbed in such a form, inflammation on the lips and skin around the lips and adverse reactions such as anorexia are associated with continuous taking. The food products of the invention enables ingestion of iron without associating such adverse effects at all.

Moreover, use of a digestion product of a mixture of pulverized soybean and pulverized carrot in the process of the invention would provide food products with nutritive elements such as protein, lipid, saccharide and carotene which are abundantly contained in soybean and carrot, in readily absorbable forms and without loss.

In addition, the food products produced from pulverized carrot according to the process of the invention are thoroughly miscible with oil and exhibit a high emulsifying activity, and thus they are also useful as an edible emulsifier.

The invention will be described below in more details with reference to examples.

Example 1

A mixture of 120 g of powders of skinned raw soybean with 240 g of hot water is boiled. 1.0 kg of raw carrot is cooked for 45 min. and then wet pulverized. To the pulverized carrot is added 1.0 kg of warm water so as to maintain the temperature at 40-45°C. The soybean mixture is blended with the carrot mixture to give a jelly mass to which a digestive enzyme is added at a ratio of 1/1000 to the substrate. The mixture is subjected to digestion for one hour, and 12 g of ferric citrate is dissolved in the digestion mixture. The resulting mass is inoculated with 300 g of Saccharomyces cerevisiae followed by growth and fermentation at 20-25°C for two hours. Before completion of the fermentation, organic acids thereby formed are neutralized with calcium carbonate to adjust the reduced pH to 5.0. Brownish black color, characteristic of ferric citrate, disappears as fermentation progresses. The culture is dried to give the desired product.

Example 2

A mixture of 60 g of powders of skinned raw soybean with 120 g of hot water is boiled. Raw carrot weighing 1.0 kg is boiled for 30 min. and then wet pulverized. To the pulverized carrot is added 1.0 kg of warm water so as to maintain the temperature at 40-45°C. The soybean mixture is blended with the carrot mixture to give a liquid mass to which is then added a digestive enzyme at a ratio of 1/1000 to the substrate. The mixture is subjected to digestion for one hour, and 12 g of ferrous lactate is dissolved in the digestion mixture. The resulting mass is inoculated with 300 g of Saccharomyces cerevisiae followed by growth and fermentation at 20-25°C for two hours. Before completion of the fermentation, organic acids thereby formed are neutralized with calcium carbonate to a pH of 5.0 The culture is dried to give the desired product.

Example 3

Powders of skinned raw soybean weighing 120 g is mixed with 360 g of hot water, and the mixture is boiled. After cooled to a temperature of 50°C, a digestive enzyme is added to the resulting mixture at a ratio of 1/50 to the substrate followed by digestion. Centrifugal separation and addition of water are repeated to produce 1000 g of the hydrolyzate. Separately, 1.0 kg of raw carrot is boiled for 30 min. and then wet pulverized. The pulverized carrot is combined with the above hydrolyzate, and further digestion is made at 45°C for one hour. In the digestion mixture is dissolved 12 g of ferrous lactate, and the resulting mixture is inoculated with 300 g of Saccharomyces cerevisiae. Growth and fermentation are carried out at 20-25°C for two hours. Before completion of the fermentation organic acids thereby formed are neutralized with calcium carbonate to adjust the reduced pH to 5. The culture is dried to give the desired product.

Example 4

1.0 kg of raw carrot is boiled for 30 min. and then wet pulverized to give a liquid mass. To the mass is added 1.0 kg of warm water so as to maintain the temperature at 40-45°C. To the resulting mixture is added amylase at a ratio of 1/1000 to the substrate. Saccharification is carried out for one hour. After inoculated with 300 g of Saccharomyces cerevisiae, 12 g of ferrous lactate is dissolved followed by growth and fermentation at 20-25°C for two hours. Before completion of the fermentation organic acids thereby formed are neutralized with calcium carbonate to adjust the reduced pH. The greenish white color of ferrous lactate is masked by color of carotene, but the acids formed are characteristically rich in lactic acid. The culture is used either as it is or after dried.

Example 5

Powder of skinned raw soybean weighing 300 g is mixed with 800 g of hot water, and the mixture is boiled for 30 min. Subsequently, while maintaining the temperature at 40-45°C, 12 g of ferric citrate is dissolved in, and a commercially available saccharifying enzyme is added at a ratio of 1/1000 to the substrate. The resulting mixture is subjected to saccharification treatment for one hour.

The treated solution is inoculated with 300 g of Saccharomyces cerevisiae while maintaining the temperature at 20-25°C followed by fermentation, for two hours. Before completion of the fermentation, pH reduced during the fermentation is neutralized with calcium carbonate, and the culture is dried to give the desired product.

Test Example

20 female volunteers were given the iron enriched food products obtained in Examples 1 and 2 and a control food product respectively at a daily dose of 1 g (containing 5 mg or more of iron) within 30 min. after every evening meal. Number of the persons with occurrence of inflammation on the lips and skin around the lips was counted by age range. Results are shown in Table 1. The control food product is a simple mixture consisting of pulverized and boiled soybean, boiled and pulverized carrot and ferrous lactate.

4

EP 0 399 040 B1

Table 1

| Tested person | Test material | | | | | | | | |
|---|---|---|---|---|---|---|---|---|---|
| | Food product of Example 1 | | | Food product of Example 2 | | | Control food product | | |
| | Day 2 | Day 4 | Day 6 | Day 2 | Day 4 | Day 6 | Day 2 | Day 4 | Day 6 |
| 15-18 year-old (female) | 0 | 0 | 0 | 0 | 0 | 0 | 0 | 1 | 2 |
| 25-30 year-old (female) | 0 | 0 | 0 | 0 | 0 | 0 | 0 | 2 | 4 |

As evidently demonstrated by the above results, whereas intake of the food product of the invention is associated with no adverse reaction at all, intake of a simple mixture of the components of the invention without yeast fermentation occasionally causes inflammation on the lips and skin around the lips.

Industrial Applicability

The iron enriched food products according to the present invention not only contain iron in readily absorbable and adverse reaction-free form but also are rich in nutritive elements such as protein, lipid, saccharide, carotene and calcium so that they are useful as food for patients, especially for iron-deficient patients.

**Claims**

1. A process for preparing an iron enriched food product which comprises hydrolyzing pulverized soybean, pulverized carrot or a mixture of the two with saccharide-decomposing enzyme and cultivating yeast in said hydrolyzate in the presence of an iron compound.

2. A process according to Claim 1 wherein pulverized and boiled soybean produced by mixing powdery raw soybean with sizes not larger than 200 mesh (74 $\mu$m) with hot water and boiling the mixture, said powdery raw soybean being obtained by finely pulverizing skinned raw soybean, or boiled and pulverized carrot produced by boiling and pulverizing the boiled carrot, or a mixture of the two is hydrolyzed with amylase, and Saccharomyces cerevisiae is cultivated in said hydrolyzate in the presence of an iron compound.

3. A process according to Claim 1 or 2 wherein the iron compound is iron citrate or iron lactate.

**Patentansprüche**

1. Verfahren zur Herstellung eines eisenangereicherten Nahrungsmittels, umfassend:

   Hydrolysieren pulverisierter Sojabohnen, pulverisierter Karotten oder eines Gemisches der beiden mit einem saccharidabbauenden Enzym und

   Züchten von Hefe in diesem Hydrolysat in Gegenwart einer Eisenverbindung.

2. Verfahren nach Anspruch 1, wobei durch Vermischen pulverförmiger roher Sojabohnen mit Größen von nicht mehr als 200 Mesh (74 $\mu$m) mit heißem Wasser und Kochen des Gemisches hergestellte pulverisierte und gekochte Sojabohnen, wobei die pulverförmigen rohen Sojabohnen durch feines Pulverisieren geschälter roher Sojabohnen hergestellt worden sind, oder durch Kochen und Pulverisieren der gekochten Karotten hergestellte gekochte und pulverisierte Karotten oder ein Gemisch der beiden mit Amylase hydrolysiert werden, und in diesem Hydrolysat in Gegenwart einer Eisenverbindung Saccharomyces cerevisiae gezüchtet wird.

3. Verfahren nach Anspruch 1 oder 2, wobei die Eisenverbindung aus Eisencitrat oder Eisenlactat besteht.

**Revendications**

5

1. Procédé de préparation d'un produit alimentaire enrichi en fer, comprenant l'hydrolyse de soja pulvérisé, de carottes pulvérisées ou d'un mélange de ces deux substances par une enzyme décomposant les saccharides et la culture d'une levure dans ledit hydrolysat en présence d'un composé du fer.

2. Procédé selon la revendication 1, dans lequel du soja pulvérisé et bouilli, produit par mélange de soja brut en poudre de granulométrie inférieure ou égale à 200 mesh (74 $\mu$m) et d'eau très chaude et par ébullition du mélange, ledit soja brut en poudre étant obtenu par pulvérisation fine de soja brut épluché, ou des carottes bouillies et pulvérisées produites par ébullition et pulvérisation des carottes bouillies ou un mélange de ces deux substances est hydrolysé par une amylase, et Saccharomyces cerevisiae est cultivé dans ledit hydrolysat en présence d'un composé du fer.

3. Procédé selon la revendication 1 ou 2, dans lequel le composé du fer est du citrate de fer ou du lactate de fer.